# EUROPÄISCHE PATENTSCHRIFT

(11) **EP 1 153 398 B1**
(45) Veröffentlichungstag und Bekanntmachung des Hinweises auf die Patenterteilung: **16.11.2005**
(21) Anmeldenummer: 00910672.5
(22) Anmeldetag: 11.02.2000
(51) Int. Cl.: G21K 1/04, A61N 5/10

(54) **IONENSTRAHL-ABTASTSYSTEM UND VERFAHREN ZUM BETRIEB DES SYSTEMS**
ION BEAM SCANNER SYSTEM AND OPERATING METHOD
SYSTEME DE BALAYAGE A FAISCEAU IONIQUE ET PROCEDE PERMETTANT DE FAIRE FONCTIONNER CE SYSTEME

(30) Priorität: 19.02.1999 DE 19907098
(43) Veröffentlichungstag der Anmeldung: 14.11.2001
(73) Patentinhaber: Gesellschaft für Schwerionenforschung mbH, 64291 Darmstadt (DE)
(72) Erfinder: KRAFT, Gerhard, D-64291 Darmstadt (DE); WEBER, Ulrich, D-64291 Darmstadt (DE)
(74) Vertreter: Boeters, Hans Dietrich
(86) Internationale Anmeldenummer: PCT/EP2000/001149
(87) Internationale Veröffentlichungsnummer: WO 2000/049624

(56) Entgegenhaltungen:
- EP-A- 0 094 278
- EP-A- 0 223 432
- EP-A- 0 417 965
- EP-A- 0 779 081
- WO-A-96/19262
- GB-A- 1 362 678

## Beschreibung

Die Erfindung betrifft ein Ionenstrahl-Abtastsystem und ein Verfahren zum Betrieb des Systems gemäß dem Oberbegriff der Ansprüche 1 und 11.

Ein derartiges System ist aus der Druckschrift US 5,585/642 bekannt und wird in der Teilchentherapie eingesetzt. Die mit einem derartigen System durchführbare Ionenstrahltherapie von Tumorgeweben zeichnet sich vor allem durch eine bessere Dosisverteilung, d.h. eine höhere Tumordosis, und eine Entlastung des gesunden Gewebes gegenüber einer Röntgentherapie aus. Diese Dosisverteilung folgt aus den physikalischen Eigenschaften von Teilchenstrahlen, die ein invertiertes Dosisprofil, d.h. ein Ansteigen der Dosis mit der Eindringtiefe, aufweist. Dadurch kann die Tumordosis über die konventionell mögliche Dosis der üblichen Bestrahlungstherapie gesteigert werden.

Um eine möglichst gute Anpassung des bestrahlten Volumens an das vorgegebene zielvolumen zu erreichen, werden in der derzeitigen klinischen Praxis Vorrichtungen zur passiven Strahlformung eingesetzt, die aber das Problem nicht zufriedenstellend lösen können. Derartige Strahlformungsvorrichtungen arbeiten mit einem divergenten Ionenstrahl, der ein größeres Gebiet als das Zielvolumen, d.h. als das Volumen des Tumors, bestrahlt, aber durch entsprechende Randbegrenzer und durch die Tumorkontur nachbildende Kompensationsformen aus Kompensationsmaterialien den divergenten Ionenstrahl auf das Tumorvolumen einengen. Derartige Anlagen und Methoden haben den Nachteil, daß eine hohe Ionenstrahlenergie für den divergenten Ionenstrahl erforderlich wird und nicht gezielt einzelne Volumenelemente eines Zielvolumens oder Tumors angesteuert werden können.

Um einzelne Volumenelemente gezielt ansteuern zu können und eine Strahlendosis für das Volumenelement optimal anpassen zu können, wurde eine Rasterscan Vorrichtung für Ionenstrahlen entwickelt. Mit dieser Vorrichtung wird das Zielvolumen in Schichten gleicher Teilchenreichweite zerlegt und ein feiner, intensitätsgesteuerter Bleistiftstrahl aus Ionen rasterförmig über die einzelnen Schichten geführt. Zusammen mit der aktiven Energie Variation durch einen Ionenbeschleuniger kann damit ein exaktes Ausleuchten in drei Dimensionen eines jeden beliebigen Zielvolumens erreicht werden.

Jedoch auch diese intensitätsgesteuerte Rasterscanvorrichtung hat erhebliche Nachteile. Zum einen ist ein aufwendiges Kontrollsystem zur Überwachung der Strahllokalisation im Mikrosekundenbereich erforderlich. Ferner besteht die Gefahr der Zerstückelung von Isoenergieschnitten durch Dichte-Inhomogenitäten. Ferner ist die Einhaltung der vorgegebenen lateralen Strahllage (Strahl Schwerpunkt) insbesondere bei der Variation von Energie und Strahlbreite während einer Behandlung problematisch. Schließlich sind die Randabfälle des Zielvolumens, die von der Breite des Strahlprofils abhängen, nachteilig für eine präzise Bestrahlung des Zielvolumens.

Diese Probleme führen dazu, daß die Einstellung und Kontrolle der Strahlparameter einer derartigen Vorrichtung ein Vielfaches der eigentlichen Patientenbestrahlung dauert. Darüber hinaus wird die Kombination von Rasterscanvorrichtung für Ionenstrahlen mit einem beweglichen drehbaren Ionenstrahlführungssystem der US-5,585,642, einem Gantry-System, wie es in Fachkreisen vorgeschlagen wird, eine erhebliche technische Herausforderung darstellen.

Bei einer Integration des Rasterscans in einem Gantry-System, wie sie aus der Druckschrift EP 0 779 081 A1 bekannt ist, werden die Kontrollen noch langwieriger und noch aufwendiger als bei einer bisher realisierten starren Strahlführung. Außerdem werden Magnete mit großen Aperturen für die Integration der vorgeschlagenen Rasterscanvorrichtung benötigt, um praxisgerechte Größen bei den Bestrahlungsfeldern zu erreichen. Zusammen mit einer schnellen Energievariation schließen die großen Aperturen dieses Lösungsvorschlags und damit die notwendige Abschirmung der Streufelder die Verwendung von supraleitenden Magneten aus. Eine Integration eines Scanner Systems führt somit zu großen Aperturen, d.h. zu großen Ablenkungsmagneten und langen raum- und kostenaufwendigen Gantry-Systemen. Bei einer Anordnung des Scannersystems hinter dem letzten Ablenkungsmagneten, also stromabwärts der Ionenstrahlführunq und eines Austrittsfensters des Ionenstrahls aus dem Führungssystem, wie es aus der Druckschrift EP 0 779 081 A2 bekannt ist, sind zwar kleine Aperturen möglich, d.h. kompakte Magnete, können eingesetzt werden, dafür ergeben sich wegen der erforderlichen Driftlänge oder der lichten Weite für einen Behandlungsraum Gantry-Radien von über 7 m. In beiden Fällen müssen demnach nachteilig Massen von mehr als 100 Tonnen millimetergenau bewegt werden.

Aufgabe der Erfindung ist es, die Nachteile im Stand der Technik zu überwinden und insbesondere im Vergleich zu der vorgeschlagenen Rasterscan Vorrichtung von Ionenstrahlen ein Ionenstrahl-Abtastsystem anzugeben, das die gegenwärtigen Schwierigkeiten überwindet.

Diese Aufgabe wird mit dem Gegenstand der unabhängigen Anspruchs 1 und 11 gelöst. Weitere Merkmale bevorzugter Ausführungsformen der Erfindung werden in den abhängigen Ansprüchen angegeben.

Zur Lösung der Aufgabe wird ein Ionenstrahl-Abtastsystem mit einer Ionenquelleneinrichtung, einem Ionenbeschleunigungsssystem und einer Ionenstrahlführung mit Ionenstrahl-Austrittsfenster für einen konvergierenden zentrierten Ionenstrahl geschaffen, mit dem ein mechanisches Ausrichtsystem für das abzutastende Zielvolumen verbunden ist. Derartige Ausrichtsysteme sind aus dem Stand der Technik bekannt und sollen gewährleisten, daß das Zielvolumen aus beliebig bestimmbaren Winkeln im Raum bestrahlt werden kann. Deshalb weist üblicherweise ein derartiges Ausrichtsystem einen Patiententrägertisch auf, der mindestens um eine Rotationsachse drehbar und in drei Verschieberichtungen bewegbar ist, die nach dem Ausrichten während der Bestrahlung üblicherweise (zum Teil) nicht mehr verändert werden.

Bei dem erfindungsgemäßen Ionenstrahl-Abtastsystem ist das Ionenbeschleunigungssystem auf eine für eine maximale Eindringtiefe notwendige Beschleunigung der Ionen einstellbar, und das Abtastsystem weist ein Energieabsorptionsmittel auf, das in dem Ionenstrahlengang zwischen Zielvolumen und Ionenstrahl-Austrittsfenster quer zum Ionenstrahlzentrum angeordnet ist und quer zum Ionenstrahlzentrum zur Variation der Ionenstrahlenergie verschieblich ist. Dazu wird erfindungsgemäß zur Tiefenmodulation des Ionenstrahls das Energieabsorbtionsmittel mittels eines Linearmotors quer zum Ionenstrahl verschoben, wobei im Zielvolumen eine in der Tiefe gestaffelte Abtastung von Volumenelementen des Zielvolumens vorteilhaft in schneller Folge durchführbar ist, wozu ein elektronisches Steuersystem für den Linearbetrieb der Absorberkeile vorgesehen ist.

Dieses Ionenstrahl-Abtastsystem, das auf einer Tiefenmodulation basiert, bietet eine vorteilhafte Verbesserung gegenüber dem Einbau und der Integration eines Rasterscansystems in ein Gantry-System. Das erfindungsgemäße System ermöglicht eine gleich gute Dosis Verteilung wie das Rasterscans System, benötigt aber ein wesentlich weniger aufwendiges Kontroll System. Darüber hinaus erlaubt es eine kompakte Bauweise auch in Zusammenwirken mit supraleitenden Magneten, so daß mit diesem System beliebige Feldgrößen realisierbar werden. Schließlich wird mit diesem System eine größere Schonung des gesunden Gewebes oberhalb eines Tumorgewebes und insbesondere eine verbesserte Haut Schonung erreicht. Darüber hinaus ist das erfindungsgemäße Ionenstrahl-Abtastsystem universell sowohl für starre Ionenstrahlführungssysteme als auch für drehbare Ionenstrahlführungssysteme einsetzbar.

Das Energieabsorptionsmittel des Ionenstrahlabtastsystems weist quer zum Ionenstrahlzentrum verschiebliche Absorberkeile auf, die mit einem leistungsstarken Linearmotor angetrieben werden, so daß eine strahlintensitätskontrollierte Tiefenrasterung vollzogen werden kann. Das Absorberkeilsystem moduliert die Eindringtiefe des Strahls durch Abbremsung, d.h., das Bragg-Maximum wird über der Tumortiefe moduliert. Die seitliche Auslenkung kann durch eine Patientenverschiebung in den zwei Richtungen beispielsweise in x- und in y-Richtung einer Ebene ausgeführt werden. Das hat den Vorteil, daß nur ein feiner Bleistiftstrahl von feststehender Energie durch die gesamte Ionenstrahlführung zu steuern ist. Die ortsfeste Lage des Strahls in diesem System kann vorteilhaft durch eine mechanisch feststehende Aperturblende garantiert werden und kann weiterhin durch kleine ortsauflösende Zähler überprüft werden. Die Strahlintensität wird mit einer einfachen Ionisationskammer meßbar, um die Strahlendosis pro Volumenelement aufzusummieren.

In einer bevorzugten Ausführungsform weist das Abtastsystem ein elektronisches Steuersystem für den Linearantrieb der Absorberkeile und eine Ionisationskammer zum Messen der Teilchenrate des Strahls auf. Die Absorberkeile werden nach Erreichen einer vorbestimmten Teilchenzahl, die von der Ionisationskammer gemessen wird, und die durchaus für den Tiefenschritt verschieden sein kann, um einen Schritt, vorzugsweise von 10 bis 100 µm, weiter zusammengefahren, so daß eine in der Tiefe gestaffelte Abtastung von Volumenelementen des Zielvolumens möglich wird.

Mit dieser Ausführungsform ist der Vorteil verbunden, daß große ortsauflösende Detektoren, die das gesamte Strahlungsfeld abchecken, nicht benötigt werden. Das bedeutet eine erhebliche Reduktion des Kontrollsystems und eine Verkleinerung des Gesamtsystems.

Theoretisch kann das Absorberkeilsystem aus einem einzigen Absorberkeil bestehen, der quer zum Ionenstrahl schrittweise bewegt wird, und aufgrund seiner zunehmenden Dicke die Eindringtiefe des Ionenstrahls in das Gewebe oder das Zielvolumen vermindert. Dadurch entsteht eine säulenförmige Abtastung des Zielvolumens. Jedoch hat ein Absorberkeilsystem, das lediglich ein Absorberkeil aufweist, den Nachteil, daß über der Breite des Ionenstrahls die Absorption variiert und damit auch die Eindringtiefe variiert. In einer bevorzugten Ausführungsform der Erfindung weist deshalb das Energieabsorptionsmittel mindestens zwei quer zum Ionenstrahlzentrum entgegengesetzt verschiebliche Absorberkeile auf. Diese beiden Absorberkeile haben den gleichen Absorberkeilwinkel, so daß beim schrittweisen Zusammenschieben der beiden Absorberkeile der Ionenstrahl immer die gleiche Dicke eines Absorbermaterials durchfahren muß. Jedoch bilden sich auch hier minimale Energieunterschiede über dem Querschnitt des Ionenstrahls aus, da der Ionenstrahl selbst konvergiert und somit an den Absorberkeilflächen beim Übergang von einem Absorberkeil zum zweiten Absorberkeil einen unterschiedlichen Querschnitte aufweist und damit über dem Querschnitt unterschiedlich absorbiert wird.

Um diesen Effekt zu vermindern, weist vorzugsweise das Energieabsorptionsmittel zwei quer zum Ionenstrahlzentrum gegeneinander verschiebliche Absorberkeilpakete auf. Bei derartigen Absorberkeilpaketen wird der Spalt zwischen zwei Absorberkeilen auf mehrere Spalte verteilt, bei denen sich die oben angegebenene nachteilige Wirkung größtenteils bei entsprechender Anordnung der Absorberkeile zueinander aufhebt und die Steigung pro Keil gegenüber einem System mit zwei gegeneinander verschieblichen Keilen vermindert wird.

In einer weiteren bevorzugten Ausführungsform der Erfindung weist das Ionenstrahlabtastsystem einen Randbegrenzer mit verschieblichen Blendenelementen zwischen dem Zielvolumen und dem Energieabsorptionsmittel auf. Eine derartige mechanische Randbegrenzung hat den Vorteil, daß steilere Randabfälle möglich werden, ohne daß ein aufwendiges Kontrollsystem erforderlich wird. Dazu weist vorzugsweise das Abtastsystems irisblendenartig einzeln verstellbare Randblenden für eine derartige Randbegrenzung des Ionenstrahl in bezug auf das Zielvolumen auf.

Um vorzugsweise das gesamte Zielvolumen zu bestrahlen, weist das Ionenstrahlabtastsystem einen Patiententisch auf, der das Zielvolumen trägt und während eines Bestrahlungsvorganges in einer Ebene quer zum Ionenstrahl in zwei Koordinatenrichtungen verschiebbar ist.

Mit dem erfindungsgemäßen Tiefenmodulationssystem wird das Zielvolumen primär in Säulen um die einzelnen Zielstrahlen zerlegt. Längs der Säule wird der Strahlweg in einzelne Positionen oder Pixel aufgeteilt, für die die Strahlbelegung bzw. die Strahlendosis vorberechnet wird. Mit einem mechanischen Abbremssystem zur Energieabsorption der Ionen, das aus einem Mehrfachkeil mit Linearantrieb besteht, wird das Bragg-Maximum des Strahls intensitätsgesteuert, ohne Unterbrechung von einem Pixel zum nächsten Pixel geführt, wenn die Teilchenbelegung der einzelnen Pixel erreicht ist. Diese Säulenaufteilung entspricht mehr den wirklichen Gegebenheiten einer Tumorbestrahlung, als die Flächenaufteilung der vorgeschlagenen Rasterscanvorrichtung, da Dichteinhomogenitäten stromaufwärts des Zielvolumens in, beispielsweise in der gesunden Gewebestruktur über einem Tumor, zu einer Verschiebung einer Säule in positiver oder negativer Richtung führen, aber nicht zu einer Unterbrechung innerhalb der Säule.

Zur Tumorbehandlung eines Patienten wird bei der erfindungsgemäßen Lösung vorteilhaft die höchste benötigte Energie für das Strahlenführungssystem vom Beschleuniger bis zum Patienten eingestellt. Diese Energieeinstellung bleibt in vorteilhafter Weise für die gesamte Behandlung konstant, da die Tiefenvariation, d.h. die Energievariation, nur durch das schnell bewegliche Absorberkeilsystem des Energieabsorptionsmittels direkt vor dem Patienten geschieht. Die Länge der einzelnen Bestrahlungssäulen hängt von der Geometrie des Zielvolumens ab. Der Dosisquerschnitt der Säulen ist ein Gauß-Profil. Die Abstände der Säulenzentren müssen kleiner sein als die halbe Halbwertsbreite der Gauß -Verteilung, um eine kontinuierliche Überlappung zu erzeugen. Um über- und unterbestrahlte Stellen auszuschließen, ist ein relativ breites Strahlprofil im Größenordnungsbereich von 10mm Durchmesser vorteilhaft. Diese im Vergleich zur vorgeschlagenen Rasterscanvorrichtung großen Strahlprofile setzen auch die Bestrahlungsdauer pro Patient bei großen Zielvolumina herab.

Da durch den Randbegrenzer einer bevorzugten Ausführungsform der Erfindung Teile der radialen Dosisverteilung der einzelnen Dosissäulen absorbiert werden können, kann trotz großer Halbwertsbreiten ein steiler Randabfall an kritischen Stellen erreicht werden.

Während das Energieabsorptionsmittel in Form einer Tiefenmodulationsvorrichtung, bzw. eines Tiefenmodulators, bzw. eines Tiefenscanners aufgrund des Antriebs des Absorberkeilsystems mittels eines luftgelagerten Linearmotors schnell und säulenförmig das Zielvolumen abtasten kann, ist für die seitliche Verschiebung des Zielvolumens in den zwei Richtungen x und y einer Ebene genügend Zeit, so daß ein Patiententisch, der das Zielvolumen trägt und in zwei lateralen Richtungen quer zum Ionenstrahl während eines Bestrahlungsvorgangs verschiebbar ist, genügend Zeit, um nach und nach Säule für Säule abzutasten und nebeneinander zu überlappen.

In einer weiteren bevorzugten Ausführungsform der Erfindung ist der Patiententisch während der Behandlung nur in einer Koordinatenrichtung verschieblich, während für die andere laterale Richtung entsprechende Ablenkungsmagnete für den Ionenstrahl vorgesehen sind, so daß der Ionenstrahl aus seiner Mittenposition am Austrittsfenster quer zur lateralen Richtung des Patiententisches ablenkbar wird. Dieses System hat den Vorteil, daß es lediglich nur noch in einer Richtung von einer mechanischen und damit langsamen Bewegung abhängt und eine Tiefenmodulation und Seitenmodulation relativ zügig durchgeführt werden kann.

Vorzugsweise ist eine Ionisationskammer zur Summation der Ionen, die in einem Volumenelement auftreffen, stromaufwärts vom Energieabsorbtionsmittel und stromabwärts vom Ionenstrahl-Austrittsfenster angeordnet. Mit dieser Anordnung kann die Bestrahlungsdosis, die als Summenzahl der Ionen, die in einem Volumenelement auftreffen, definiert ist, vorteilhaft bestimmt werden.

In einer weiteren bevorzugten Ausführungsform der Erfindung weist das Ionenstrahlabtastsstem zusätzlich zu einem in lateraler Richtung verschiebbaren Patiententisch ein Gantry-System auf, das quer zur lateralen Bewegungsrichtung des Patiententisches um eine Gantryrotationsachse drehbar ist. Durch die langsame Pendelbewegung des Gantry-Systems über das Bestrahlungsfeld werden die einzelnen Säulen nebeneinandergesetzt und damit vorteilhaft die zweite Dimension der tumorkonformen Bestrahlung ausgeführt.

Bei dem bevorzugten Ionenstrahl-Abtastsystem mit Gantry-System wird der Ionenstrahl in der Gantryrotationsachse dem Gantry-System zugeführt und mittels Magnetoptiken unter einstellbaren Winkeln von 0 bis 360° in einer Ebene orthogonal zur Gantryrotationsachse auf ein Zielvolumen ausgerichtet. Der Ionenstrahl schneidet dabei die Gantryrotationsachse in einem Isozentrum des Gantry-System. Dabei weist das Gantry-System einen lateral in Richtung der Gantryrotationsachse verschieblichen Zielvolumenträger auf, der stromaufwärts des Isozentrums angeordnet ist. Das Energieabsorptionsmittel ist stromaufwärts in Radialrichtung des Gantry-Systems angeordnet. Durch die erfindungsgemäße Tiefenmodulationsvorrichtung wird eine Volumenelementrasterung in Tiefenrichtung erreicht, durch das Gantry-System eine Drehwinkel-Volumenelementrasterung in Seitenrichtung und durch den lateral verschieblichen Zielvolumenträger eine Volumenelementrasterung in Längsrichtung definiert, so daß beliebig geformte Zielvolumen durch die Kombination dieser drei Rastermittel volumenelementweise rasterbar sind.

Bei dieser bevorzugten Ausführungsform der Erfindung ist es wichtig, daß das Isozentrum der Gantrybewegung stromabwärtsvon, bzw. hinter dem Bestrahlungsvolumen liegt. Eine leichte Kegelförmigkeit des Bestrahlungsvolumens kann durch eine entsprechende Wichtung der Pixel bei einem dichten Netz der radialen Stützpunkte berücksichtigt werden, ohne daß ein Verlust an Homogenität auftritt.

Die Winkeldifferenz der exzentrischen Bestrahlung durch die Gantryrotation hat den entscheidenden Vorteil einer weiteren Absenkung der Dosisbelastung im Eingangskanal und damit einer Absenkung der Dosisbelastung des gesunden Gewebes über dem Tumorgewebe. Da sich das Gantry-System wegen der großen Masse nur kontinuierlich oder in kleinen Schritten bewegen kann, muß die angebotene Teilchenfluenz höher als die pro Säule benötigte Fluenz liegen. Das bedeutet, die Bestrahlung jeder einzelnen Säule vollzieht sich während einer kurzen Winkelbewegung des Gantry-Systems. Die dritte Koordinate der Bestrahlung erfolgt bei dieser bevorzugten Ausführungsform durch eine langsame, schrittweise Verschiebung des liegenden Patienten auf dem Zielvolumenträger durch die Bestrahlungsapparatur in Richtung der Gantryrotationsachse. Dabei werden vorzugsweise Geschwindigkeiten von weniger als 1 cm/s eingehalten und sind ausreichend für tolerable Bestrahlungszeiten von Patienten.

In einer weiteren bevorzugten Ausführungsform der Erfindung bleibt der Zielvolumenträger während der Behandlung unbewegt und der Ionenstrahl wird durch die Umlenkmagnete während der Bestrahlung in der Gantry-Ebene abgelenkt. Damit wird, statt die Patientenliege zu verschieben, der Ionenstrahl durch Variation des Magnetfeldes im letzten Umlenkmagneten in der Gantry abgelenkt. Es ergibt sich damit vorteilhaft folgende Abfolge der notwendigen Bewegung in drei Freiheitsgraden: die Strahlablenkung mit der höchsten Geschwindigkeit geschieht mit dem Energieabsorbtionsmittel, bzw. Tiefenmodulator. Eine mittlere Geschwindigkeit (z.B. alle 1 bis 2 s um 4 mm), wird der Strahl in der Gantry-Ebene durch die Umlenkmagneten zur nächsten Säule geführt. Die langsamste Bewegung ist die Gantryrotation, die nach der Bestrahlung einer Säulenreihe durch die Drehung des Gantry-Systems auf die nächste Säulenreihe geführt wird. Der Vorteil bei diesem Ionenstrahl-Abtastsystem ist es, daß der Patient nicht bewegt werden muß und das Gantry-System während der Bestrahlung nicht hin- und herschwingen muß, sondern schrittweise gedreht werden kann.

Gegenüber herkömmlichen Systemen erfordert die variable Ablenkung des Strahls in der Gantry-Ebene nur einen relativ geringen Zusatzaufwand, da die Umlenkmagnete sowieso notwendig sind. Die Veränderung der Ablenkung des Strahls erfolgt langsam (im Sekundentakt) und somit werden auch keine hohen Anforderungen an die Magnetnetzteile und das Kontrollsystem gestellt. Als Kontrollvorrichtung für die Ablenkungsvariation des Strahls genügt eine einfache Drahtkammer, die mit einer relativ geringen Wiederholungsrate ausgelesen werden muß. Die Kontrollelektronik für diese langsame Abtasttechnik der vorliegenden Erfindung ist bei weitem nicht so aufwendig, wie die vorgeschlagene, konkurrierende, schnelle Rasterscantechnik, bei der die Strahlpositionen alle 100 µs zu messen sind, um den Strahl verfolgen und kontrollieren zu können.

In einer bevorzugten Ausführungsform des Ionenstrahl-Abtastsystems mit Gantry-System weist das Energieabsorptionsmittel tangential zum Drehkreis des Gantry-Systems verschiebliche Absorberteile auf. Diese Ausführungsform wird dadurch erreicht, daß das Energieabsorptionsmittel unmittelbar an dem Gantry-System fixiert wird, nämlich stromabwärts von dem Austrittsfenster des Ionenstrahls. Auch in dieser Ausführungsform des Ionenstrahl-Absorptionssystems mit Gantry-System können statt einen Absorberkeil mindestens zwei tangential zum Drehkreis des Gantry-Systems entgegengesetzt verschiebliche Absorberkeile vorgesehen werden oder radial gestaffelte tangential zum Drehkreis des Gantry-Systems verschiebliche Absorberkeilpakete angeordnet werden.

In einer weiteren bevorzugten Ausführungsform des Ionenstrahl-Abtastsystems mit Gantry-System ist ein zentraler Bereich des Zielvolumens um mindestens 1/5 des Gantrysystemradius stromaufwärts des Isozentrums angeordnet, so daß das Zielvolumen selbst nicht im Isozentrum liegt. Die Vorteile dieser Ausführungsform wurden bereits oben im Detail geschildert. Dabei ist hervorzuheben, daß ein optimaler Abstand zwischen Zielvolumen und Isozentrum einstellbar ist, um die Belastung des gesunden Gewebes im Eingangskanal der Bestrahlung gering zu halten.

Bei Verfahren zum Ionenstrahlabtasten mittels einer Ionenquelleneirichtung, einem Ionenbeschleunigungssystem und einer Ionenstrahlführung mit Ionenstrahl-Austrittsfenster für einen konvergierenden, zentrierten Ionenstrahl und einem mechanischen Ausrichtsystem für das abzutastende Zielvolumen werden folgende Verfahrensschritte durchgeführt:
- Einstellen des Ionenbeschleunigungssystems auf eine für eine maximale Eindringtiefe notwendige Beschleunigung der Ionen,
- Erfassen der Ionenstrahlintensität,
- Querverschieben eines Energieabsorptionswittels mit unterschiedlicher Materialstärke zur Tiefenmodulation des Ionenstrahls,
- Aufsummieren der Strahlungsionen eines Volumenelementes eines Zielvolumens bis zu einer vorgegebenen Strahlungsdosis,
- Ändern der Eindringtiefe des Ionenstrahls mittels Querverschiebung des Energierbsorptionsmittels nach Erreichen der vorgegebenen Bestrahlungsdosis des Volumenelementes zur Bestrahlung eines nächsten stromaufwärts liegenden Volumenelements.

Mit diesem Verfahren ist der Vorteil verbunden, daß die Beschleunigung der Ionen in dem Ionenbeschleunigungssystem nur einmal festzulegen ist und während der gesamten Behandlungsphase beibehalten werden kann. Die Tiefenmodulation der Bestrahlung wird ausschließlich durch ein Energieabsorptionsmittel, das stromaufwärts des zu bestrahlenden Zielvolumens angeordnet ist, und stromabwärts des Ionenstrahl-Austrittsfensters liegt, durchgeführt. Da es außer dem Energieabsorbtionsmittel, bzw. dem Tiefenmodulator kein weiteres Material im Strahlengang gibt, bevor das Gewebe erreicht wird, ist die nukleare Fragmentierung minimal und unabhängig von der Eindringtiefe, da die Gesamtabsorption durch den Tiefenmodulator plus Gewebetiefe insgesamt konstant bleibt. Somit wird ein konstantes Bragg-Profil über der Zielvolumentiefe erreicht. Eine Verwendung von Spezialfiltern, auch Ripple-Filtern, zur Vergleichmäßigung des Bragg-Profils, wie es herkömmliche Verfahren einsetzen, ist bei dem erfindungsgemäßen Verfahren entbehrlch.

Die Bewegung der Absorberkeile wird für die Tiefenmodulation von der Intensität des einkommenden Strahls nach der berechneten Vorlage der Dosisverteilung geregelt. Dabei ist die Länge jeder Bestrahlungssäule in einzelne Bildpunkte aufgeteilt und der Strahl, d.h. das Bragg-Maximum des Strahls, wird vorteilhaft von einem Pixel, bzw. Bildpunkt, zum nächsten verschoben, wenn die erforderliche Teilchenzahl erreicht ist. Somit bietet das erfindungsgemäße Verfahren eine größtmögliche Sicherheit für den Patienten und eine hohe Präzision bei der Bestrahlung von Tumorgeweben sowie eine minimale Belastung des stromaufwärts liegenden gesunden Gewebes.

In einer Durchführung des Verfahrens fährt ein elektronisches Steuersystem für den Linearantrieb der Absorberkeile diese um einen Schritt weiter zusammen, nachdem es die Teilchenrate des Strahles mittels einer Ionisationskammer gemessen hat, und eine vorbestimmte Teilchenanzahl, die für jeden Tiefenschritt unterschiedlich sein kann, erreicht worden ist. Somit erfolgt eine in der Tiefe gestaffelte Abtastung von Volumenelementen des Zielvolumens. Vorzugsweise ist die Schrittweite, mit der die Absorberkeile weiter zusammengefahren werden, zwischen 10 und 100 µm.

Die gemessene Intensität pro Volumenelement liegt zwischen 10⁶ und 10⁸ absorbierten Ionen während des Abrasterns des Zielvolumens. Dabei kann das Zielvolumen kontinuierlich fortschreitend abgetastet werden, indem während der Tiefenmodulation gleichzeitig in den anderen beiden Richtungen der Patiententisch oder die Gantry oder beides bewegt wird. Zwar erfolgt aufgrund der Tiefenmodulation die Abtastung des Zielvolumens in Tiefenrichtung immer säulenförmig, jedoch können diese Säulen bei einer kontinuierlich fortschreitenden Abtastung zickzackförmig verschoben sein.

In einer anderen Durchführung des Verfahrens schreitet die Abtastung des Zielvolumens schrittweise voran. Dieses schrittweise Voranschreiten ist insbesondere vorteilhaft, wenn die Bewegungsabläufe mit unterschiedlichen Geschwindigkeiten aufgrund von unterschiedlichen Massen, die zu bewegen sind, ablaufen.

Entsprechend kann die Abtastung des Zielvolumens in Tiefenrichtung kontinuierlich und in Seiten- und Längsrichtung schrittweise erfolgen oder die Abtastung des Zielvolumens in Tiefenrichtung und in Seitenrichtung kontinuierlich und in Längsrichtung schrittweise erfolgen. Bei einer bevorzugten Durchführung des Verfahrens wird das Ionenstrahl-Abtastsystem unter Verwendung eines Gantry-Systems betrieben. Dazu werden folgende Verfahrensschritte durchgeführt:
1 . Anordnen des Zielvolumens stromaufwärts des Isozentrums,
2. Volumenelementrastern in Tiefenrichtung mittels eines Energieabsorptionsmittels, das stromaufwärts in Radialrichtung des Gantry-Systems angeordnet ist,
3. Volumen element rastern in Seitenrichtung mittels Drehwinkeländerung des Gantry-Systems und
4. Volumenelementrastern in Längsrichtung mittels Rasterverschiebung des Zielvolumenträgers.

Dieses Verfahren hat den Vorteil, daß der Zielvolumenträger oder der Patiententisch lediglich in eine Richtung während der Bestrahlung bewegt werden muß, während die anderen beiden Richtungen einerseits durch die Tiefenmodulation mit Hilfe des Energieabsorptionsmittels erreicht wird, und die andere durch Dreh- oder Pendelbewegung des Gantry-Systems durchgeführt wird.

Während für das Verschieben des Absorberkeilsystems des Energieabsorptionsmittels relativ geringe Massen mit einem Linearmotor zu bewegen sind, erfolgt die Drehbewegung eines Gantry-Systems entsprechend langsamer, während ein Verschieben eines Patiententisches aufgrund seiner geringeren Masse gegenüber einen Gantry-System, aber größeren Masse gegenüber dem Energieabsorptionsmittel mit seiner Trägheit zwischen diesen beiden Vorrichtungen liegt.

Bei eine weiteren Verfahren kann der Patient in völliger Ruhe auf dem Patiententisch liegen, wenn ein Gantry-System Verwendung findet, bei dem ein Zielvolumenträger vor der Behandlung ausgerichtet wird und während der Bestrahlung unbeweglich bleibt und der Ionenstrahl mittels der letzten Gantryumlenkmagnete in der Gantry-Ebene zur Volumenelementrasterung in Längsrichtung abgelenkt wird.

Weitere Vorteile, Merkmale und Anwendungsmöglichkeiten der Erfindung werden nun anhand von Ausführungsbeispielen näher erläutert.
Fig. 1 zeigt eine schematische Darstellung einer ersten Ausführungsform der Erfindung,
Fig. 2 zeigt das Prinzip der Überlagerung von zueinander verschobenen Tiefendosisprofilen,
Fig. 3 zeigt das Prinzip der säulenförmigen Abtastung eines Zielvolumens mit der Ausführungsform nach Fig. 1,
Fig. 4 zeigt die perspektivische Ansicht eines Energieabsorptionsmittels in Form eines einzelnen Absorberkeils,
Fig. 5 zeigt den Querschnitt eines Energieabsorbermittels mit zwei Absorberkeilen,
Fig. 6 zeigt den Querschnitt eines Energieabsorptionsmittels mit Absorberkeilpaketen aus jeweils fünf Absorberkeilen,
Fig. 7 zeigt einen Randbegrenzer in Ruhestellung,
Fig. 8 zeigt einen Randbegrenzer mit zwei Blenden in Betriebsposition,
Fig. 9 zeigt eine Prinzipsskizze eines luftgelagerzen Linearmotors,
Fig. 10 zeigt eine Prinzipskizze einer zweiten Ausführungsform der Erfindung,
Fig. 11 zeigt eine Prinzipskizze der Anordnung eines Zielvolumens in der Ausführungsform nach Fig. 10 oberhalb des Isozentrums,
Fig. 12 zeigt eine Prinzipskizze einer dritten Ausführungsform der Erfindung.

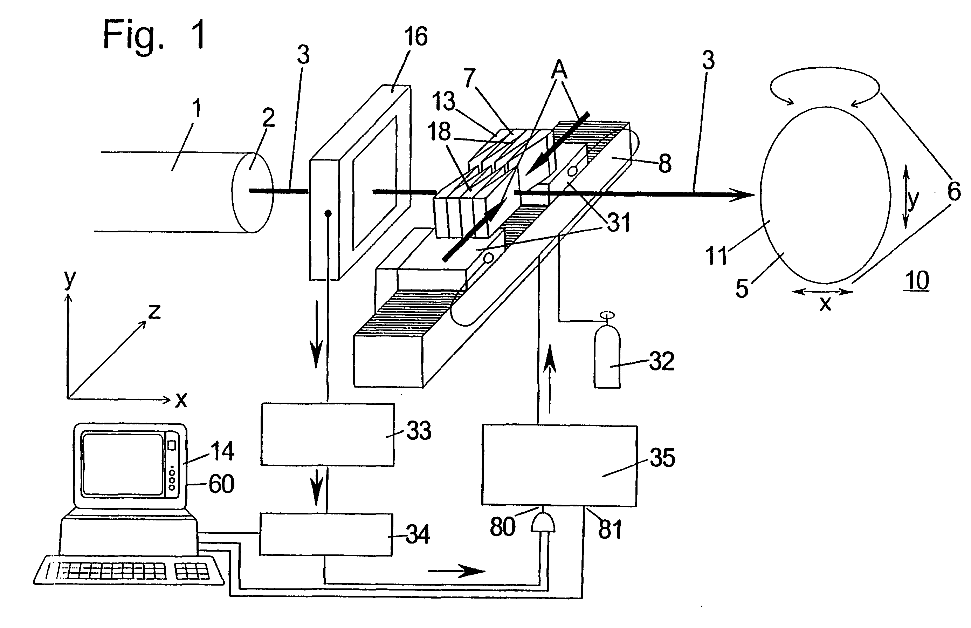

Fig. 1 zeigt eine schematische Darstellung einer ersten Ausführungsform eines Ionenstrahl-Abtastsystems, das eine nichtgezeigte Ionenquelleneinrichtung, ein nichtgezeigtes Ionenbeschleunigungssystem und eine Strahlführung 1 mit einem Ionenstrahl-Austrittsfenster 2 für einen konvergierenden zentrierten Ionenstrahl 3 umfaßt. Zu dem Ionenstrahl-Abtastsystem gehört ein nichtgezeigtes mechanisches Ausrichtsystem für das abzutastende Zielvolumen 5.

Das Ausrichtsystem ermöglicht es, das Zielvolumen mindestens um eine Achse zu drehen und in Richtung der drei Raumkoordinaten zu verschieben, so daß das Zielvolumen aus einem beliebig wählbaren Winkel im Raum von dem Ionenstrahl bestrahlt werden kann. Eine derartige Ausrichtung erfolgt vor der eigentlichen Bestrahlung des Zielvolumens mit Ionen.

Mit dem nicht gezeigten Ionenbeschleunigungssystem wird zunächst eine für eine maximale Eindringtiefe notwendige Beschleunigung der Ionen eingestellt. Diese Energie wird während der gesamten Behandlung nicht geändert und bewirkt, daß der Ionenstrahl bis zu dem tiefsten Punkt des Zielvolumens das Gewebe durchdringen kann. Durch ein Energieabsorptionsmittel 7, das in dem Ionenstrahlengang zwischen Zielvolumen 5 und Ionenstrahl-Austrittsfenster 2 quer zum Ionenstrahlzentrum angeordnet ist und quer zum Ionenstrahlzentrum zur Variation der Ionenstrahlenergie in Pfeilrichtung A verschieblich ist, wird das Bragg-Maximum von der maximalen Eindringtiefe durch zunehmende Energieabsorption zu niedrigeren Eindringtiefen verschoben. Dieser Vorgang kann kontinuierlich oder schrittweise durchgeführt werden, so daß eine Tiefenmodulation oder eine Tiefenabtastung stattfindet, die im folgenden auch Tiefenscan genannt wird. Die Änderung der Energieabsorption wird mittels des Energieabsorptionsmittels 7 dadurch erreicht, daß ein Linearmotor 8 eine Querverschiebung in Pfeilrichtung A von paarweise gegenüberstehenden Absorberkeilen 13 aufeinander zu bewirkt, so daß ein in der Tiefe gestaffeltes Abtasten von Volumenelementen des Zielvolumens in schneller Folge durchführbar wird.

Dazu sind die Läufer 31 des Linearmotors 8 luftgelagert und werden von einer Preßluftflasche 32 mit Druckluft versorgt. Für ein schrittweises Zusammenfahren der Absorberkeile 13 werden die Motorströme über eine Schrittmotorsteuerung 35, die eine Leistungsstufe aufweist, gesteuert.

Um die Anzahl der Ionen zu messen und aufzusummieren, bis eine vorbestimmte Ionenstrahldosis pro Volumenelement des Zielvolumens erreicht ist und bis der Linearmotor 8 um einen weiteren Schritt die Absorberkeile 13 vorzugsweise um 10 bis 100 µm zusammenfahren kann, wird mit der Ionisationskammer 16 über einen Stromverstärker mit nachgeschaltetem Spannungs-FrequenzKonverter 33 eine Impulssteuerung mit TTL-Impulsen angesteuert, deren Frequenzproportional zur einfallenden Ionenrate onen/sec) ist. Sobald die ausreichende Dosis für ein Volumenelement erreicht ist, was einer bestimmten TTL-Impulszahl entspreicht, löst die Impulssteuerung mit einem Steuerimpuls an die Schrittmotorsteuerung den nächsten Schritt aus. Dieser Ablauf wiederholt sich, bis ein Tiefenscan abgeschlossen ist.

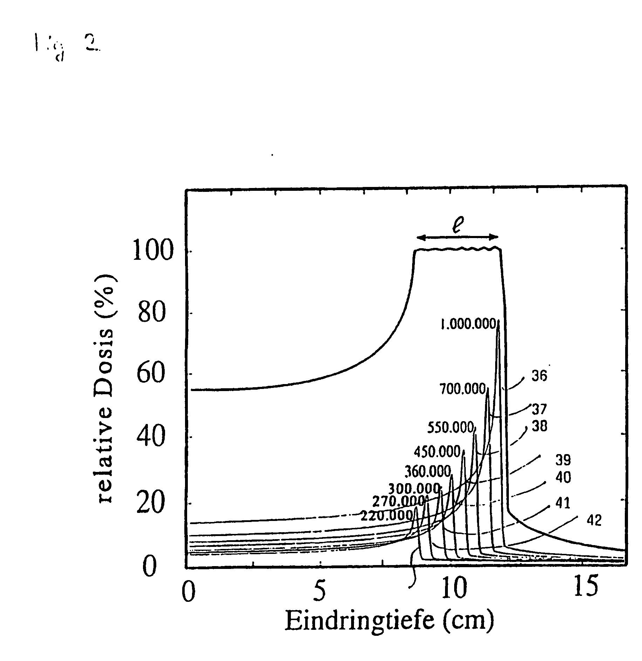

Fig. 2 zeigt das Prinzip der Überlagerung von zueinander verschobenen Tiefendosisprofilen. Durch den Tiefenscanner werden die einzelnen Bragg-Kurven 36 bis 44 von einer zur nächsten Absorberstellung um jeweils 4,3 mm verschoben. Die Höhe der Bragg-Kurven ist durch die Teilchenanzahl gegeben, die bei entsprechender Absorberstellung auf das Target auftrifft. Wenn die Teilchenzahlen vorher korrekt berechnet wurden, ergibt die Überlagerung der Bragg-Kurven die gewünschte verbreiterte Bragg-Spitze, die der Ausdehnung des Zielvolumens in der Tiefe entspricht. Entsprechend ist auf der Abszisse die Eindringtiefe in cm angegeben und auf der Ordinate die relative Dosis in %.

Deutlich ist an diesem Beispiel erkennbar, daß bei der Aufsummation der Bragg-Kurven das Zielvolumen mit 100 % relativer Dosis bestrahlt wird, während das darüberliegende gesunde Gewebe unter 60 % der Strahlenbelastung ausgesetzt ist, und das darunterliegende Gewebe sogar weit unter 20 % der Strahlendosis aufnehmen muß. Damit wird deutlich, welch großer Vorteil gegenüber Röntgenstrahltherapien die Ionenstrahltherapie aufweist.

Die Fig. 2 soll das Prinzip der Überlagerung lediglich verdeutlichen. Bei einem realen Tiefenscan werden mehrere tausend Bragg-Kurven mit viel kleineren Abständen überlagert. Dieses ermöglicht eine quasi kontinuierliche Bewegung der Antriebe des Energieabsorptionsmittels 7 und gewährleistet somit eine gleichmäßige Überlagerung, so daß auf konventionelle Ripple-Filter bei Einsatz des Ionenstrahl-Abtastsystems nach dieser Ausführungsform der Erfindung verzichtet werden kann.

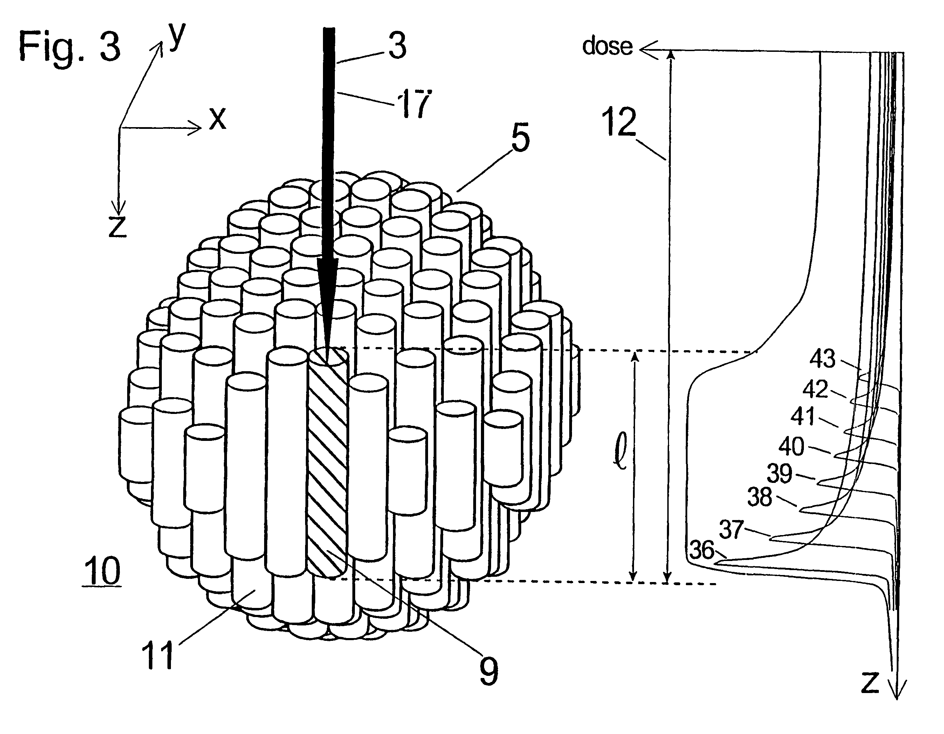

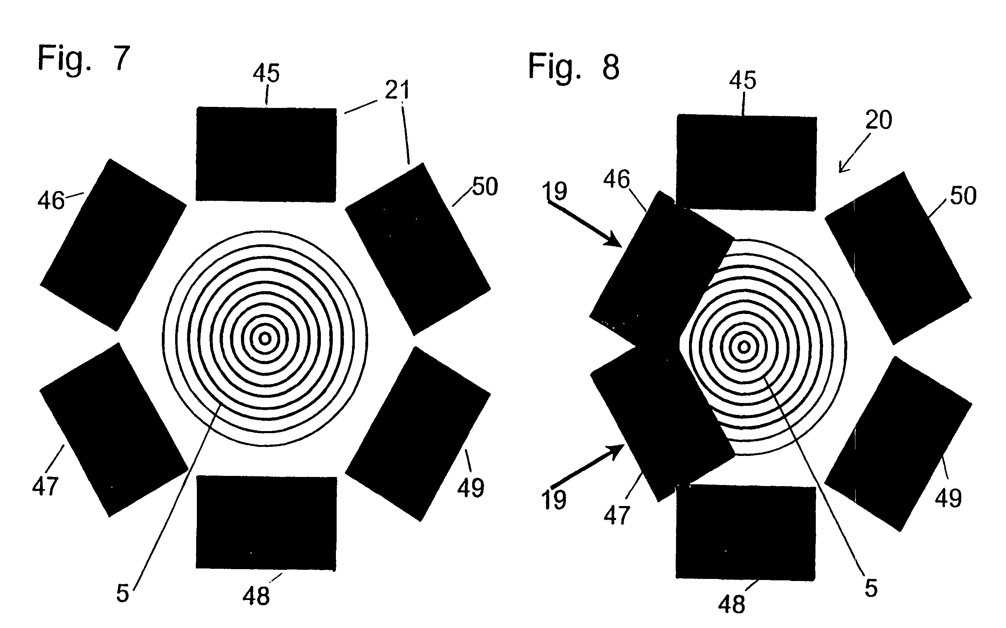

Fig. 3 zeigt das Prinzip der säulenförmigen Abtastung eines Zielvolumens mit der Ausführungsform nach Fig. 1. Bei dieser Ausführungsform wird das Zielvolumen in Pfeilrichtung X und Y mechanisch verschoben, während der Ionenstrahl 3 einer starren Ionenstrahlführung seine zentrale Richtung beibehält. Durch die Tiefenmodulation oder den Tiefenscan verursacht durch das Energieabsorptionsmittel werden die Volumenelemente 9 des Zielvolumens 5 säulenförmig abgetastet, wobei die Ausdehnung 1 der verbreiterten Bragg-Spitze aufgrund der Summation der Bragg-Kurven 36 bis 43 der Länge der Säule bzw. der Tiefe des Zielvolumens an dieser Stelle entspricht. Wie Fig. 3 deutlich zeigt, kann ein gesundes Gewebe 10 von einer Bestrahlung weitestgehend verschont bleiben, während das Tumorgewebe die unterschiedlichsten Formen einnehmen kann, so daß lediglich die Tiefenmodulation der Ausdehnung des Tumorgewebes in einer Koordinatenrichtuing, beispielsweise entlang der z-Koordinate ,folgen muß.

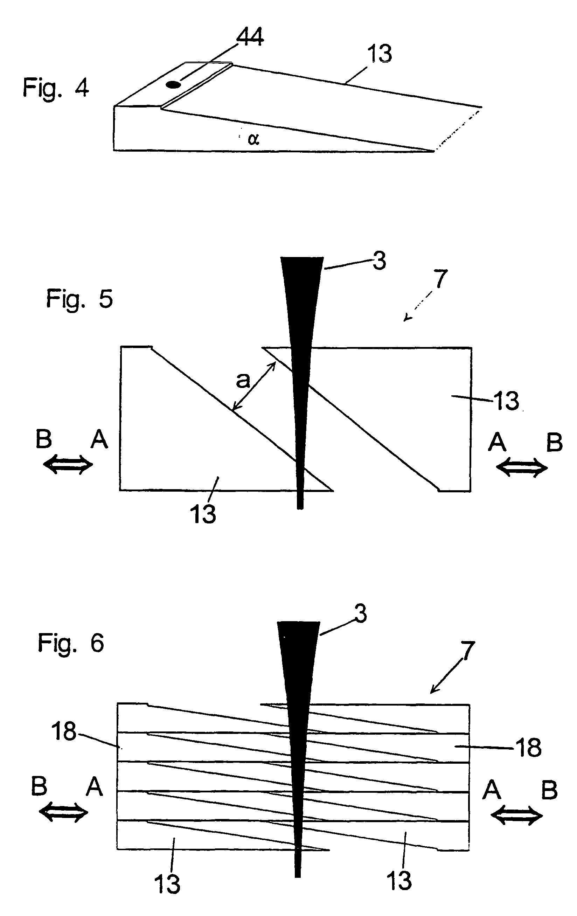

Fig. 4 zeigt die perspektivische Ansicht eines Energieabsorptionsmittels in Form eines einzelnen Absorberkeils. Dieser Absorberkeil ist ein Einzelkeil aus einem Absorberkeilpaket, wie es in Fig. 6 gezeigt wird. Der Absorberkeilwinkel α liegt zwischen 6 und 10° und ist in diesem Ausführungsbeispiel auf 8,765° ± 0,01 festgelegt. Die Länge des Absorberkeils beträgt zwischen 100 und 150mm und ist in diesem Ausführungsbeispiel auf 120 ± 0,02mm festgelegt. Die größte Dicke des Absorberkeils beträgt zwischen 15 und 30mm und ist in diesem Ausführungsbeispiel auf 19 ± 0,01mm angesetzt. Der quaderförmige Abschluß des Absorberkeils dient dazu, ihn in einem Paket, wie es in Fig. 6 gezeigt wird, zu stapeln. Der quaderförmige Abschluß hat die Abmessungen 15 x 15mm² bis 30 x 30mm² und eine Länge von 40 bis 60 mm, die in diesem Beispiel 50mm beträgt. Der Quaderquerschnitt ist der größten Absorberkeiltiefe von 19 mm angepaßt und beträgt in diesem Beispiel 20 x 20mm². Eine zentrale Bohrung 44 ermöglicht das Zusammenfügen mehrerer Einzelkeile zu Absorberkeilpaketen, wie sie in Fig. 6 gezeigt werden.

Fig. 5 zeigt den Querschnitt eines Energieabsorptionsmittels mit zwei Absorberkeilen 13, die in Pfeilrichtung A aufeinander zu oder in Pfeilrichtung B auseinander fahrbar sind. Werden diese zwei Absorberkeile auseinandergefahren, so ergibt sich ein großer Abstand a zwischen den schrägen Absorberkeilflächen. Aufgrund des konvergierenden zentrierten Ionenstrahls 3 wird deutlich, daß der Ionenstrahlbereich auf der rechten Seite des Strahls ein dickeres Absorbermaterial zu durchstrahlen hat, als auf der linken Seite des Strahls. Somit ist die Eindringtiefe in das Zielvolumen über den Querschnitt des Ionenstrahls gesehen unterschiedlich. Um dieses auszugleichen und zu minimieren, wird in Fig. 6 der Querschnitt eines Energieabsorptionsmittels 7 mit Absorberkeilpaketen 18 aus jeweils fünf Absorberkeilen 13 gezeigt. Deutlich ist zu erkennen, daß die rechte Seite des konvergenten Ionenstrahls kaum mehr Absorbermaterial zu durchdringen hat, als die linke Seite. Somit ist eine Tiefenmodulation mit Hilfe von Absorberkeilpaketen 18 einer Tiefenmodulation aus zwei Absorberkeilen 13, wie in Fig. 5 gezeigt, vorzuziehen.

Vorzugsweise bestehen die Absorberkeile 13 aus mehreren Plexiglaskeilen. Aufeinandergesetzt besitzen sie als Absorberkeilpaket 18 die gleiche Wirkung wie zwei Absorberkeile 13 der Fig. 5, jedoch mit unterschiedlicher Steigung. Eine große Steigung ist bei zwei Absorberkeilen, wie in Fig. 5 notwendig, um bei der erreichbaren Beschleunigung der Absorberkeile 13 einen ausreichenden Absorbereffekt zu erzielen, diese große Steigung wird in Fig. 6 auf eine Mehrzahl von Absorberkeilen mit kleiner Steigung verteilt.

Der Vorteil von mehreren aufeinandergesetzten gegenüber zwei dicken Absorberkeilen 13 liegt darin, daß der Strahl in den Absorberkeilen unvermeidlich aufgestreut wird, und bei den zwei dicken Absorberkeilen ein Teil des Strahls durch mehr Material als ein anderer Teil des Strahls läuft. Dadurch erzeugt man eine undefinierte Energieverteilung des Strahls, die zu vermeiden wäre. Die Absorberkeile 13 werden mit Aluminiumwinkeln auf dem Linearmotoren 8 befestigt. Die Verschraubungen sind spielfrei und präzise.

Das Plexiglas als Material der Absorberkeile 13 hat den Vorteil, präzise bearbeitbar zu sein und aus radiologischer Sicht dem Material Wasser recht ähnlich zu sein. Demnach verhält sich für einen Ionenstrahl aus Kohlenstoff eine 1 cm dicke Plexiglasschicht genauso wie eine 1,15 cm dicke Wasserschicht. Bei einer Verwendung von 2 x 4 Absorberkeilen mit einem Steigungswinkel von α = 8,765° ergibt sich ein Vorschub durch die Motoren um 1 mm eine Verdickung des Absorbers um eine wasseräquivalente Strecke von 1,42 mm Wasser.

Das Ionenstrahl-Abtastsystem gemäß der vorliegenden Erfindung stellt hohe Anforderungen an das System zur Tiefenmodulation und die Beweglichkeit des Energieabsorptionsmittels 7. Das Energieabsorptionsmittel, bzw. der Tiefenscanner soll mit einem Schwerionenstrahl ein glattes Tiefendosisprofil mit einer typischen Ausdehnung von 2 bis 15 cm in der Tiefe erzeugen. Dazu werden Absorberkeile verwendet, die so verschoben werden können, daß die Strecke, die der Strahl durchläuft, verändert wird. Der Tiefenscanner variiert während der Strahlextraktion die Absorberdicke in genau definierter Weise. Die Schwerionenstrahlen, die durch unterschiedlich dicke Absorptionseinstellungen gelaufen sind, werden dabei überlagert und erzeugen in der Addition das gewünschte Tiefendosisprofil.

Damit in der Überlagerung keine Abweichungen von der Vorgabe auftreten, muß bei jeder Absorberkeildickeneinstellung eine genau definierte Ionenstrahlmenge oder Teilchenmenge auf das Target treffen. Das bedeutet, wenn die Absorberkeile zulange oder zu kurz in einer bestimmten Stellung verharren, ergeben sich Abweichungen vom gewünschten Tiefen-Dosis-Profil. Um derartige Abweichungen zu vermeiden, muß der Ionenstrahlstrom als Funktion der Zeit gemessen werden und je nach gemessener Anzahl der Ionen sind die Absorberkeile ständig mit hoher Präzision und unterschiedlicher Schrittgeschwindigkeit zu verschieben.

Da der Ionenstrahlstrom insbesondere bei Schwerionen, die durch ein Ionenbeschleunigersystem, vorzugsweise einem Synchrotron, beschleunigt wurden, nicht konstant ist, sondern sehr starke Fluktuationen zeigt, hat dieser unregelmäßige Ionenstrahlstrom zur Folge, daß die Absorberkeile 13 auch mit sehr unregelmäßiger Geschwindigkeit teilweise ruckartig verschoben werden müssen. Dies stellt eine extreme Anforderung an Präzision und Dynamik des Antriebs und der Steuerung für ein derartiges Energieabsorptionsmittel 7 dar, so daß vorzugsweise folgende Parameter einzuhalten sind:

| | |
|---|---|
| Extraktionszeit | 2 - 4 s |
| Tiefendosisprofil | 2 - 15 cm |
| Maximale Geschwindigkeit | 1 - 2 m/s |
| Beschleunigung | 20 - 30 m/s² |
| Genauigkeit | 100 - 200 µm |

Verwendet werden dazu Absorberkeilpakete, wie sie in Fig. 6 gezeigt werden.

Um den Vorschub der Absorberkeile möglichst synchron zur gemessenen Ionenstrahlintensität zu führen, muß die Meß- und Steuerungselektronik relativ schnell reagieren. Die Reaktionszeit, also die Summe der Verzögerungszeitkonstanten von der Ionisationskammer 16, dem Stromverstärker 33 und der Schrittmotorsteuerung 35, wie in Fig. 1 gezeigt werden, ist folglich kleiner als 1 ms.

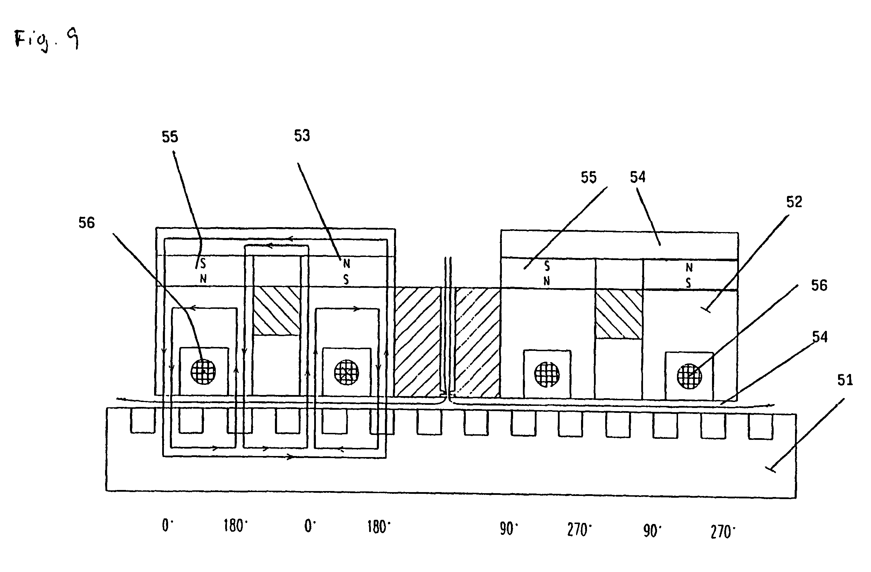

Eine geringere Anforderung an die Dynamik wird für den Randbegrenzer 20 gefordert. Bild 7 zeigt einen Randbegrenzer in Ruhestellung. Er besteht in diesem Beispiel aus sechs einzelnen rechteckigen Wolframplatten 45 bis 50, die individuell in Richtung auf das Zentrum aus ihrer in Fig. 7 gezeigten Ruhestellung geschoben werden können. Dazu sind die Wolframplatten 45 bis 50 in ihrer Höhe versetzt angeordnet, so daß sie sich beim Zusammenschieben nicht behindern. Nähert sich der Ionenstrahl dem Rand eines Tumorgewebes, bzw. Zielvolumens 5, so kann die entsprechende Randblende 19, wie in Fig. 8 gezeigt, verschoben werden, so daß eine scharfe Randbegrenzung für den relativ breiten Ionenstrahl ermöglicht wird. Im Prinzip kann bereits durch drei in der Höhe gestaffelte Blenden aus rechteckigen Wolframplatten ein beliebiger Rand des Gewebes begrenzt werden, jedoch haben sechs Platten den Vorteil der größeren Variationsmöglichkeit.

Fig. 9 zeigt eine Prinzipskizze eines luftgelagerten Linearmotors 8, auf dem jeweils ein Absorberpaket 18 eines Energieabsorptionsmittels 7 befestigt werden kann. Ein derartiger Linearmotor ist ein permanent-erregter Zweiphasen-Reluktanzmotor und besteht aus zwei Baueinheiten, dem ortsfesten Stator 51 und einem luftgelagerten beweglichen Teil dem Läufer 52.

Ein Läufer beinhaltet mindestens zwei Magnetsysteme 53 und 54 mit je einem Permanentmagneten 55 als Konstantflußquelle. Durch einen Stromfluß in den Wicklungen 56 kann der magnetische Fluß in den Schenkeln gesteuert werden, das bedeutet, in einem Schenkel wird ein Fluß verstärkt und in dem zugehörigen anderen Schenkel geschwächt. Eine Kraftwirkung in Bewegungsrichtung resultiert aus der Energieänderung des magnetischen Feldes im Luftspalt zwischen Stator 51 und Läufer 52. Werden die beiden Wicklungen der Magnetsysteme 53 und 54 mit seitlich versetzten sinusförmigen Strömen betrieben, wird ein Gleichlaufverhalten bzw. eine bestimmte Position für jedes Stromverhältnis erreicht. Das Läuferelement ist in X-Richtung angeordnet, so daß lineare Bewegungen erfolgen können. Das Statorelement 51 besteht aus strukturierten Weicheisenstreifen, die auf Stahlkörpern, Mineralguß, natürliches Hartgestein oder auch auf leichte Glasfaser oder Kohlenstoffaserhohlkörper aufgeklebt werden.

Zur Erreichung einer aerostatischen Luftlagerung ist jeder Läufer 52 mit Luftlagerdüsen ausgestattet. Aufgrund elektromagnetischer Kraftwirkung zwischen Läufer 52 und Stator 51 ist die Luftlagerung stark vorgespannt, so daß bei Betriebsdruck der Luftspalt sehr konstant ist. Die Lebensdauer ist vorteilhafterweise prinzipiell unbegrenzt, denn Reibung und Verschleiß treten nicht auf. Schmiermittel sind auch nicht erforderlich. Das erleichtert die vorzugsweise Anwendung in medizinischen Bereichen.

Für eine Ausführungsform des erfindungsgemäßen Ionenstrahl-Abtastsystems wurde der Linearmotor so ausgelegt, daß sich zwei Motoren auf demselben Stator in entgegengesetzter Richtung in Pfeilrichtung A, wie in Fig. 1 gezeigt, bewegen. Dazu hat der Stator die Maße 64 mm x 64 mm x 1 m Länge, wovon nur etwa 50 cm Länge für den Tiefenscanner benötigt werden. Ferner besitzt der Stator eine Lamellenstruktur mit einer Periode von 1,28 mm. Die Motoren sind luftgelagert und arbeiten im Prinzip wie eine Magnetschwebebahn. Die technischen Daten der Motoren sind:

| | |
|---|---|
| Masse | 700 g |
| Maximale Haltekraft | 100 N |
| Maximale Geschwindigkeit | 2 m/s |
| Maximale Beschleunigung | 40 m/s² |
| Positioniergenauigkeit | 10 µm |
| Wiederholgenauigkeit | 3 µm |
| Führungsgenauigkeit | 5 µm |
| Luftdrucklagerung | 3,5 bar |

Die Schrittmotoransteuerung 35, wie sie in Fig. 1, Position 35 gezeigt, wird, ist für einen Tiefenscanner bzw. einen Tiefenmodulator vorteilhaft geeignet. Die Schrittmotorsteuerung 35 hat eine einfache effektive Schnittstelle, die im wesentlichen nur aus 2 TTL-Eingängen 80, 81 besteht, die einen Schrittbefehl oder einen Richtungsbefehl geben. Bei einem Schrittbefehl werden mit jedem einlaufenden TTL-Impuls an einem ersten Eingang 80 die Motorströme der Leistungsstufe so geändert, daß sich die Motoren gegenläufig um 20µm in diesem Ausführungsbeispiel weiterbewegen. für einen Richtungsbefehl bestimmt der Pegel (TTL) an dem zweiten Eingang 81 die Richtung, nämlich auseinanderlaufend oder zusammenlaufend.

Die Ionisationskammer 16, wie sie aus in Fig. 1 gezeigt wird, gibt die Anzahl der Teilchen (Schwerionen) an, die pro Sekunde vom Beschleuniger extrahiert werden. Diese Größe unterliegt starken zeitlichen Schwankungen und muß deshalb in Echtzeit gemessen werden. Eingesetzt wird hierzu eine Parallelplatten-Transmissions-Ionisationskammer mit ca. 1cm Gasstrecke (Stickstoff-CO₂-Gemisch 80 % : 20 %), die bei 1600 V betrieben wird. Der Strom, der am Ausgang der Kammer gemessen werden kann, ist bei feststehender Teilchenenergie proportional zum Strahlstrom. Bei typischen Strahlströmen des Beschleunigers liegen die Ströme aus der Ionisationskammer im Bereich von µA.

Die Ansprechgeschwindigkeit des Detektors wird durch die Driftzeit der ionisierten Detektorgasmoleküle (Ionenrümpfe) in der Ionisationskammer 16 begrenzt und hat eine Verzögerungskonstante von ca. 100 µs.

Die Meßelektronik im Stromverstärkerblock 33 wandelt den Strom aus der Ionisationskammer in eine proportionale Frequenz von TTL-Impulsen um. Aus dem Stromsignal wird ein Spannungssignal im Voltbereich erzeugt. Durch eine Amplitudenfrequenzumwandlung werden aus dem Spannungssignal TTL-Impulse erzeugt, deren Frequenz proportional zur Spannung ist. Dabei entsprechen 4 MHz etwa 10 V.

Bei gegebener Verstärkung des Stromverstärkers 33 der Fig. 1 entspricht demnach ein TTL-Impuls einer bestimmten Ladung, die in der Ionisationskammer erzeugt wurde. Diese wiederum wurde von einer bestimmten Teilchenzahl erzeugt. Die Anzahl der erzeugten TTL-Impulse ist also proportional zur Anzahl der Schwerionen, die durch die Ionisationskammer 16 fliegen. Der Proportionalitätsfaktor läßt sich experimentell auf ± 3 % genau bestimmen und hängt von der Teilchenenergie und der Verstärkung ab.

Die Impulssteuerung 34 in Fig. 1 ist im Prinzip ein variabler Impulsratenumsetzer. Die Steuerung hat einen Speicher, der in mehrere Bereiche eingeteilt werden kann. Über den angeschlossenen PC kann vor der Bestrahlung in die einzelnen Bereiche jeweils eine Zahlenreihe geladen werden. Die Anzahl der Zahlen entspricht der Anzahl der Positionen, die die Linearmotoren für einen tiefen Scan einnehmen sollen. In der Praxis sind dies einige tausend Positionen pro Bereich.

Wenn die Bestrahlung gestartet wird, zählt die Impulssteuerung die Impulse, die von der Meßelektronik kommen, und gibt ihrerseits einen Impuls an die Schrittmotorsteuerung 35 aus, wenn die Impulszahl für die erste Position erreicht wird. Dann wird der Zähler auf 0 zurückgesetzt und wiedergezählt, bis die Pulszahl für die zweite Position erreicht wird, worauf wieder ein TTL-Impuls ausgegeben wird. Dieses wiederholt sich, bis die letzte Position erreicht wurde. Dann gibt die Impulssteuerung ein Stop-Signal an die Beschleuniger, woraufhin die Strahlextraktion in wenigen µs abgebrochen wird.

Direkt im Anschluß daran kann der nächste Speicherbereich mit einer anderen Zahlenreihe aktiviert werden, um einen anderen Tiefenscan zu fahren. Der Rechner 60 dient zum Laden der Zahlenreihen in die Pulssteuerung. Außerdem kann der Rechner über eine Indexkarte die Schnittmotorsteuerung 35 direkt ansteuern. Damit können vor dem eigentlichen Tiefenscan die Motoren automatisch in ihre Referenzposition oder Startposition bewegt werden.

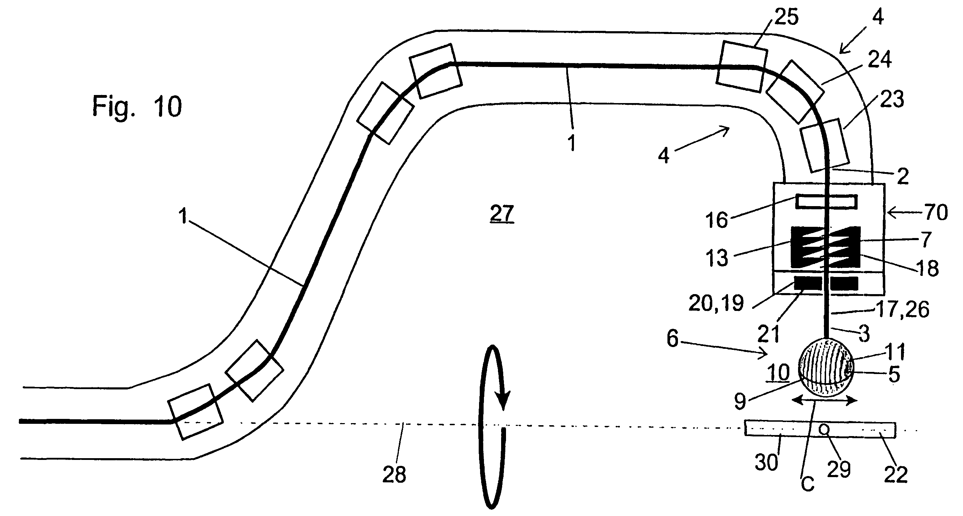

Fig. 10 zeigt eine zweite Ausführungsform der Erfindung. Der Tiefenscanner 70 entspricht dabei dem Tiefenscanner in Bauform und Wirkungsweise der Fig. 1. Jedoch wird in dieser bevorzugten Anwendung ein Gantry-System eingesetzt, das den Ionenstrahl 3 um eine Gantryrotationsachse 28 rotieren lassen kann. Mit dem Tiefenmodulator oder Tiefenscanner 70 kann das Zielvolumen 5 in Säulenform abgetastet werden und durch Drehen des Gantry-Systems um wenige Winkelgrade kann das Zielvolumen seitlich abgetastet werden. Ein besonderer Vorteil dieser Anordnung ist die Möglichkeit, das Zielvolumen oberhalb des Isozentrums 29 des Gantry-Systems 27 anzuordnen. Damit wird der Einlaufkanal für den Ionenstrahl stromaufwärts divergent, das bedeutet, daß sowohl die Haut eines Patienten als auch das gesunde Gewebe weniger belastet wird, weil die Ionenstrahl-Bestrahlung im Bereich oberhalb des Zielvolumens 5auf ein größeres Volumen verteilt wird. Durch den Einsatz des Gantry-Systems nach Abbildung 10 muß der Zielvolumenträger 30 nur noch in eine Richtung bewegt werden, vorzugsweise in Richtung der Gantryrotationsachse 28, wie es die Pfeilrichtungen C zeigen.

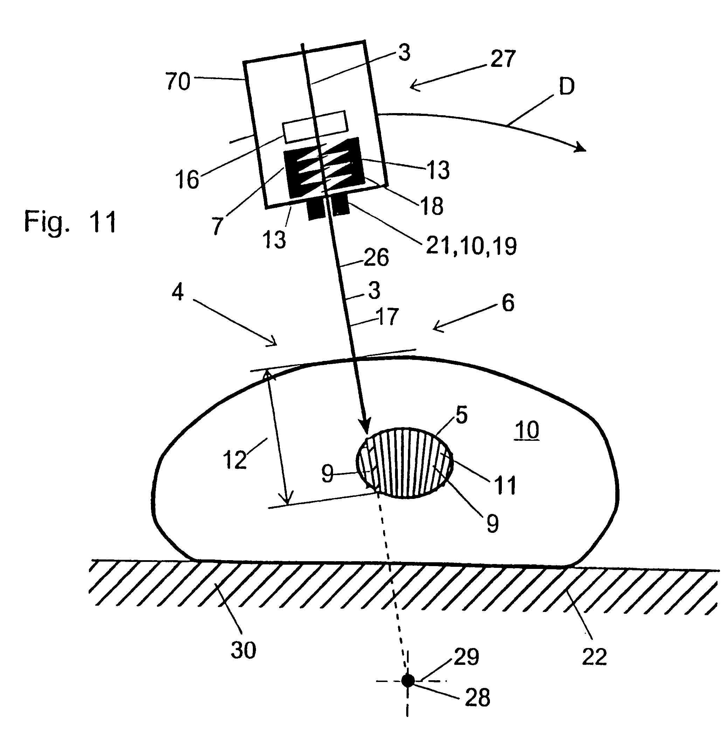

Fig. 11 zeigt eine Prinzipskizze der Anordnung eines Zielvolumens 5 in der Ausführungsform nach Fig. 10 oberhalb des Isozentrums 29. Deutlich erkennbar ist in dieser Prinzipskizze die Gantryrotationsrichtung D, in der das Zielvolumen 5 nacheinander säulenförmig mit Hilfe des Tiefenscanners 70 tiefengescannt wird. Für jedes Volumenelement 9 wird die Anzahl der Ionen in der Ionisationskammer 16 gemessen und in der Nähe der Ränder des Tumorgewebes kann mit Hilfe der Randbegrenzung 20 ein scharfer Randabfall, falls erforderlich, eingestellt werden. Deutlich erkennbar ist auch, daß sich die Bestrahlung des gesunden Gewebes 10 über dem Tumorgewebe auf ein größeres Volumen verteilt, so daß die Strahlenbelastung dews gesundes Gewebes verringert wird.

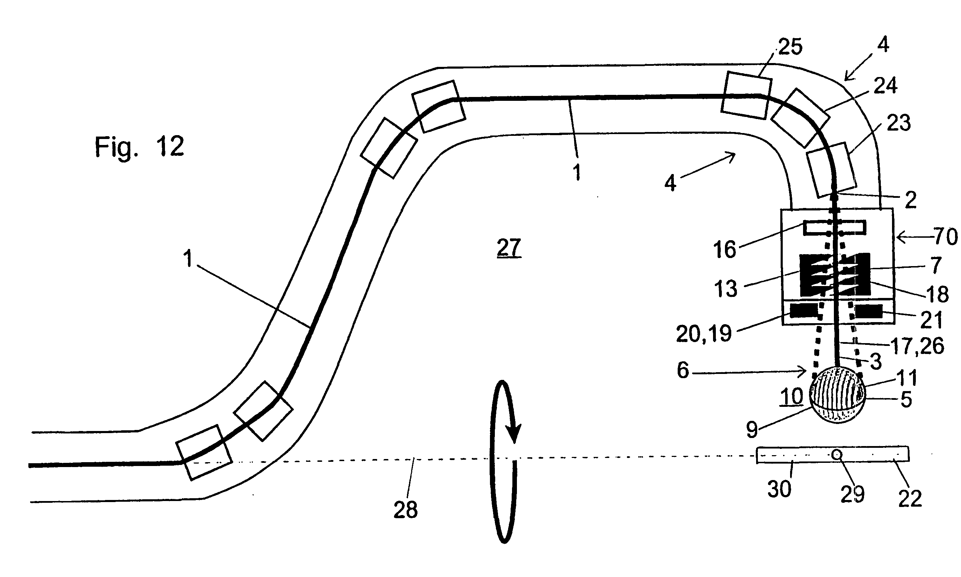

Während bei diesem System, das in den Figuren 10 und 11 dargestellt wird, der Zielvolumenträger 30 zur vollständigen Bestrahlung des Zielvolumens in Längsrichtung bewegt werden muß, zeigt Fig. 12 eine Prinzipskizze einer dritten Ausführungsform der Erfindung, bei der nach dem Ausrichten des Targetvolumens in Relation zu dem Isozentrum keine weitere mechanische Bewegung des Zielvolumens erforderlich ist. Bei der Ausführungsform nach Fig. 12 wird anstelle des Zielvolumenträgers der Ionenstrahl mit Hilfe der Ablenkungsmagnete 23, 24 und 25 der Ionenstrahlführung in dem Gantry-System in der Strahlführungsebene des Gantry-Systems abgelenkt, was keinen erheblich größeren Aufwand für die Ströme in den Ablenkungsmagneten bedeuten, zumal eine Neukonstruktion der Ablenkungsmagnete nicht erforderlich ist, da der Ionenstrahl in Richtung des Dipolspaltes der letzten Ablenkungsmagnete abgelenkt würde. Bei diesem System kommt der gleiche Tiefenscanner 70, wie bei der ersten und zweiten Ausführungsform der Erfindung nach Fig.1 und Fig. 10 zum Einsatz.

## Patentansprüche

1. Ionenstrahl-Abtastsystem mit einer Ionenquelleneinrichtung, einem Ionenbeschleunigersystem, das auf eine für eine maximale Eindringtiefe notwendige Beschleunigung der Ionen einstellbar ist, und einer Ionenstrahlführung (1) mit Ionenstrahl-Austrittsfenster (2) für einen konvergierenden zentrierten Ionenstrahl (3), einem mechanischen Ausrichtsystem (4) für das abzutastende Zielvolumen (5) und ein quer zum Ionenstrahlzentrum verschiebliches Energieabsorbtionsmittel (7), **dadurch gekennzeichnet, dass**
das Abtastsystem (6) als Energieabsorptionsmittel (7) Absorberkeile (13) aufweist und in dem Ionenstrahlengang zwischen Zielvolumen (5) und Ionenstrahl-Austrittsfenster (2) quer zum Ionenstrahlzentrum angeordnet ist und einen leistungsstarken Linearmotor (8) zum Antrieb der Absorberkeile (13), sowie ein elektronisches Steuersystem (14, 34) für den Linearbetrieb der Absorberkeile (13) aufweisend eine Ionisationskammmer (16) zum Messen der Teilchenrate des Strahls stromaufwärts der Absorberkeile (13) zur strahlintensitäts-kontrollierten Tiefen-Rasterung unter Querverschiebung des Energieabsorptionsmittels (7) aufweist, so daß eine in der Tiefe gestaffelte Abtastung von Volumenelementen (9) eines Tumorgewebes (11) als Zielvolumen (5) in Folge durchführbar ist.

2. Ionenstrahl-Abtastsystem nach Anspruch 1, **dadurch gekennzeichnet, daß** das Abtastsystem ein elektronisches Steuersystem (14, 34) für den Linearantrieb der Absorberkeile (13) aufweist und eine Ionisationskammer (16) zum Messen der Teilchenrate des Strahles umfasst und die Absorberkeile (13) nach Erreichen einer vorbestimmten Teilchenzahl, die für jeden Tiefenschritt verschieden sein kann, um einen Schritt, vorzugsweise von 10 µm bis 100 µm weiter zusammenfährt, so daß eine in der Tiefe gestaffelte Abtastung von Volumenelementen (9) des Zielvolumens (5) möglich ist.

3. Ionenstrahl-Abtastsystem nach Anspruch 1 oder Anspruch 2, **dadurch gekennzeichnet, daß** das Energieabsorptionsmittel (7) mindestens zwei quer zum Ionenstrahlzentrum entgegengesetzt verschiebliche Absorberkeile (13) auf weist.

4. Ionenstrahl-Abtastsystem nach einem der vorhergehenden Ansprüche, **dadurch gekennzeichnet, daß** das Energieabsorptionsmittel (7) zwei quer zum Ionenstrahlzentrum (17) gegeneinander verschiebliche Absorberkeilpakete (18) aufweist.

5. Ionenstrahl-Abtastsystem nach einem der vorhergehenden Ansprüche, **dadurch gekennzeichnet, daß** das System einen Randbegrenzer (20) mit verschieblichen Blendenelementen (21) zwischen dem Zielvolumen (5) und dem Energieabsorptionsmittel (7) aufweist.

6. Ionenstrahl-Abtastsystem nach einem der vorhergehenden Ansprüche, **dadurch gekennzeichnet, daß** das Abtastsystem irisblendenartig einzeln verstellbare Randblenden (19) zur teilweisen Randbegrenzung des Ionenstrahls (3) in Bezug auf das Zielvolumen aufweist.

7. Ionenstrahl-Abtastsystem nach einem der vorhergehenden Ansprüche, **dadurch gekennzeichnet, daß** es einen Patiententisch (22) aufweist, der das Zielvolumen (5) trägt und der während eines Bestrahlungsvorganges in einer Ebene quer zum Ionenstrahl (3) in zwei Koordinateneinrichtungen verschiebbar ist.

8. Ionenstrahl-Abtastsystem nach einem der vorhergehenden Ansprüche, **dadurch gekennzeichnet, daß** es einen Patiententisch (22) aufweist, der das Zielvolumen (5) trägt und der während eines Bestrahlungsvorganges in einer lateralen Richtung quer zum Ionenstrahl (3) verschiebbar ist und Ablenkmagnete (23, 24, 25) aufweist, welche den Ionenstrahl (1) aus seiner Mittenposition am Austrittsfenster (2) quer zur lateralen Richtung des Patiententisches (22) ablenkt.

9. Ionenstrahl-Abtastsystem nach einem der vorhergehenden Ansprüche, **dadurch gekennzeichnet, daß** es eine Ionisätionskammer (16) zur Messung der Strahlintensität aufweist, die stromaufwärts vom Energieabsorptionsmittel (7) angeordnet ist.

10. Ionenstrahl-Abtastsystem nach einem der vorhergehenden Ansprüche, **dadurch gekennzeichnet, daß** es einen Patiententisch (22) aufweist, der das Zielvolumen (5) trägt und während einer Bestrahlung in einer lateralen Richtung quer zum Ionenstrahl (3) verschiebbar ist und ein Gantry-System (27) aufweist, das quer zur lateralen Bewegungsrichtung des Patiententisches (22) um eine Gantryrotationsachse drehbar ist.

11. Ionenstrahl-Abtastsystem mit Gantry-System (27) zum Ausrichten eines Ionenstrahls (3) auf ein Zielvolumen (5) nach Anspruch 10, wobei der Ionenstrahl (3) in der Gantryrotationsachse (28) dem Gantry-System (27) zugeführt und mittels Magnetoptiken (23, 24, 25) unter einstellbaren Winkeln von 0 bis 360° in einer Ebene orthogonal zur Gantryrotationsachse (28) auf ein Zielvolumen (5) ausrichtbar ist, so daß der Ionenstrahl (3) die Gantryrotationsachse (28) in einem Isozentrum (29) des Gantry-Systems (27) schneidet, wobei das Gantry-System (27) einen lateral in Richtung der Gantryrotationsachse (28) verschieblichen Zielvolumenträger (30) aufweist, das Zielvolumen (5) stromaufwärts des Isozentrums (29) angeordnet ist und ein Energieabsorptionsmittel (7), das stromaufwärts in Radialrichtung des Gantry-Systems (27) angeordnet ist, eine Volumenelementrasterung in Tiefenrichtung das Gantry-System (27) eine Drehwinkel - Volumenelementrasterung in Seitenrichtung und der lateral verschiebliche Zielvolumenträger (30) eine Volumenelementrasterung in Längsrichtung definieren und beliebig geformte Zielvolumen (5) durch Kombination dieser drei Rastermittel volumenelementweise rasterbar ist, wobei das Energieabsorptionsmittel (7) quer zum Ionenstrahlzentrum verschiebliche Absorberkeile (13), einen leistungsstarken Linearmotor (8) zum Antrieb der Absorberkeile (13) sowie ein elektronisches Steuersystem (14, 34) für den Linearbetrieb der Absorberkeile (13) aufweist.

12. Ionenstrahl-Abtastsystem mit Gantry-System nach Anspruch 10 oder 11, **dadurch gekennzeichnet, daß** der Zielvolumenträger (30) während der Bestrahlung unbewegt bleibt, und Umlenkmagnete (23, 24, 25) den Ionenstrahl (3) während der Bestrahlung in der Gantry-Ebene ablenken.

13. Ionenstrahl-Abtastsystem mit Gantry-System nach einem der Ansprüche 10 bis 12, **dadurch gekennzeichnet, daß** das Energieabsorptionsmittel (7) tangential zum Drehkreis des Gantry-Systems (27) verschiebliche Absorberkeile (13) aufweist.

14. Ionenstrahl-Abtastsystem mit Gantry-System nach einem der Ansprüche 10 bis 13, **dadurch gekennzeichnete daß** das Energieabsorptionsmittel (7) mindestens zwei tangential zum Drehkreis des Gantry-Systems (27) entgegengesetzt verschiebliche Absorberkeile (13) auf weist.

15. Ionenstrahl-Abtastsystem mit Gantry-System nach einem der Ansprüche 10 bis 14, **dadurch gekennzeichnete daß** das Energieabsorptionsmittel (7) radial gestaffelte tangential zum Drehkreis des Gantry-Systems (27) verschiebliche Absorberkeilpakete (18) aufweist.

16. Ionenstrahl-Abtastsystem mit Gantry-System nach einem der Ansprüche 13 bis 15, **dadurch gekennzeichnet, daß** ein zentraler Bereich des Zielvolumens (5) um mindestens ein Fünftel des Gantry-Systemradius stromaufwärts des Isozentrums (29) angeordnet ist, so daß das Zielvolumen (9) selbst nicht im Isozentrum (29) liegt.

## Claims

1. Ion beam scanning system having an ion source device, an ion accelerator system which can be set to an acceleration of the ions required to obtain a maximum depth of penetration and an ion beam guidance system (1) comprising an ion beam outlet window (2) for a converging centred ion beam (3), and a mechanical alignment system (4) for the target volume (5) to be scanned, and energy absorption means (7) that can be displaced transverse to the centre of the ion beam,
**characterized In that**
the scanning system (6) comprises absorber wedges (13) as energy absorption means (7) that are arranged in the path of the ion beam between the target volume (5) and the ion beam outlet window (2) transverse to the centre of the ion beam and a high-performance linear motor (8) for driving the absorber wedges (13), and an electronic control system (14, 34) for the linear drive of the absorber wedges (13) including an ionisation chamber (16) for measuring the particle rate of the beam upstream of the absorber wedges (13) so that beam-intensity-controlled depth-scanning can be carried out in order to vary the energy of the ion beam, with the result that it is possible to carry out, in the target volume (5), depth modulation of the ion beam (1), which is effected by means of a linear motor with transverse displacement of the energy absorption means (7), with depth-staggered scanning of volume elements (9) of a tumour tissue (11) as target volume (5) in succession.

2. Ion beam scanning system according to claim 1, **characterized in that** the scanning system comprises an electronic control system (14, 34) for the linear drive of the absorber wedges (13) and includes an ionisation chamber (16) for measuring the particle rate of the beam and moves the absorber wedges (13) closer together by a step, preferably of from 10 µm to 100 µm, when a predetermined particle count has been reached, which particle count may be different for each depth step, so enabling depth-staggered scanning of volume elements (9) of the target volume (5).

3. Ion beam scanning system according to claim 1 or claim 2, **characterized in that** the energy absorption means (7) comprise at least two absorber wedges (13) that can be displaced in opposite directions transverse to the centre of the ion beam.

4. Ion beam scanning system according to any one of the preceding claims, **characterized in that** the energy absorption means (7) comprise two absorber wedge assemblies (18) that can be displaced in opposite directions transverse to the centre (17) of the ion beam.

5. Ion beam scanning system according to any one of the preceding claims, **characterized in that** the system comprises an edge-delimitation device (20) having displaceable shutter elements (21) between the target volume (5) and the energy absorption means (7).

6. Ion beam scanning system according to any one of the preceding claims, **characterized in that** the scanning system comprises edge shutters (19) that can be adjusted separately in the manner of an iris diaphragm in order to delimit some of the edge of the ion beam (3) with respect to the target volume.

7. Ion beam scanning system according to any one of the preceding claims, **characterized in that** it comprises a patient table (22) that carries the target volume (5) and that can be displaced in a plane transverse to the ion beam (3) in two directions of co-ordinates during an irradiation procedure.

8. Ion beam scanning system according to any one of the preceding claims, **characterized in that** it comprises a patient table (22) that carries the target volume (5) and that can be displaced in a lateral direction transverse to the ion beam (3) during an irradiation procedure and has deflection magnets (23, 24, 25) that deflect the ion beam (3) from its central position at the outlet window (2) transverse to the lateral direction of the patient table (22).

9. Ion beam scanning system according to any one of the preceding claims, **characterized in that** it has an ionisation chamber (16) for measuring the beam intensity, which is arranged upstream of the energy absorption means (7).

10. Ion beam scanning system according to any one of the preceding claims, **characterized in that** it comprises a patient table (22) that carries the target volume (5) and that can be displaced in a lateral direction transverse to the ion beam (3) during irradiation, and has a gantry system (27) that can be rotated about a gantry axis of rotation transverse to the lateral direction of movement of the patient table (22).

11. Ion beam scanning system having a gantry system (27) for aligning an ion beam (3) with a target volume (5) according to claim 10, **characterized in that** the ion beam (3) is supplied to the gantry system (27) in the gantry axis of rotation (28) and can be aligned with a target volume (5) by means of magneto-optics (23, 24, 25) at adjustable angles of from 0 to 360° in a plane orthogonal to the gantry axis of rotation (28) so that the ion beam (3) intersects the gantry axis of rotation (28) at an isocentre (29) of the gantry system (27), wherein the gantry system (27) comprises a target volume carrier (30) that can be displaced laterally in the direction of the gantry axis of rotation (28), the target volume (5) is arranged upstream of the isocentre (29) and energy absorption means (7), which are arranged radially upstream of the gantry system (27), define volume element scanning in the depth direction, the gantry system (27) defines angular volume element scanning in the lateral direction and the laterally displaceable target volume carrier (30) defines volume element scanning in the longitudinal direction, and target volumes (5) of any shape can be scanned by volume element by a combination of those three scanning means, wherein energy absorption means (7) comprises absorber wedges (7) that can be displaced transverse to the centre of the ion beam, a high-performance linear motor (8) for driving the absorber wedges (13), and an electronic control system (14, 34) for the linear drive of the absorber wedges (13).

12. Ion beam scanning system having a gantry system according to claim 10 or 11, **characterized in that** the **t**arget volume carrier (30) remains stationary during irradiation and deflection magnets (23, 24, 25) deflect the ion beam (3) in the gantry plane during irradiation.

13. Ion beam scanning system having a gantry system according to any one of claims 10 to 12, **characterized in that** the energy absorption means (7) comprise absorber wedges (13) that can be displaced tangential to the circle of rotation of the gantry system (27).

14. Ion beam scanning system having a gantry system according to any one of claims 10 to 13, **characterized in that** the energy absorption means (7) comprise at least two absorber wedges (13) that can be displaced in opposite directions tangential to the circle of rotation of the gantry system (27).

15. Ion beam scanning system having a gantry system according to any one of claims 10 to 14, **characterized in that** the energy absorption means (7) comprise absorber wedge assemblies (18) that can be displaced in a radially staggered manner tangential to the circle of rotation of the gantry system (27).

16. Ion beam scanning system having a gantry system according to any one of claims 13 to 15, **characterized in that** a central region of the target volume (5) is arranged upstream of the isocentre by at least one fifth of the radius of the gantry system, so that the target volume (9) itself does not lie in the isocentre (29).

## Revendications

1. Système de balayage à faisceau ionique avec un dispositif à source d'ions, un accélérateur d'ions, qui peut être réglé selon une accélération d'ions nécessaire pour atteindre une pénétration de rayonnement maximale, et un guidage de faisceau ionique (1) comportant une fenêtre de sortie de faisceau ionique (2) pour atteindre un faisceau ionique centré convergent (3), un système d'alignement mécanique (4) destiné au volume cible à balayer (5) et un moyen d'absorption d'énergie (7) mobile transversalement par rapport au centre du faisceau ionique, **caractérisé en ce que** :
le système de balayage (6) comporte des pièces absorbantes de forme conique (13) en tant que moyen d'absorption d'énergie (7) et est disposé transversalement par rapport au centre du faisceau ionique dans le trajet du faisceau ionique entre le volume cible (5) et la fenêtre de sortie de faisceau ionique (2), et comporte un moteur linéaire (8) performant destiné à entraîner les pièces absorbantes de forme conique (13) ainsi qu'un système de commande électronique (14, 34) permettant l'entraînement linéaire des pièces absorbantes de forme conique (13) comportant une chambre d'ionisation (16) dans le but de mesurer le taux de particules du faisceau diffusé en amont des pièces absorbantes de forme conique (13) par rapport à l'exploration en profondeur commandée en rapport avec l'intensité du faisceau lors du déplacement transversal du moyen d'absorption d'énergie (7), de sorte qu'un balayage avec des échelons dans la profondeur puisse être exécuté successivement sur des éléments du volume (9) d'un tissu tumoral (11) en tant que volume cible (5).

2. Système de balayage à faisceau ionique selon la revendication 1, **caractérisé en ce que** le système de balayage comporte un système de commande électronique (14, 34) permettant l'entraînement linéaire des pièces absorbantes de forme conique (13) et contient une chambre d'ionisation (16) dans le but de mesurer le taux de particules du faisceau et, après l'atteinte d'un nombre de particules prédéterminé qui peut être différent en fonction de chaque pas de la profondeur, entraîne les pièces absorbantes de forme conique (13) au pas suivant, de préférence de 10 µm à 100 µm, de manière à permettre un balayage échelonné dans la profondeur des éléments de volume (9) du volume cible (5).

3. Système de balayage à faisceau ionique selon la revendication 1 ou la revendication 2, **caractérisé en ce que** le moyen d'absorption d'énergie (7) comporte au moins deux pièces absorbantes de forme conique (13) mobiles opposées l'une à l'autre transversalement par rapport au centre du faisceau ionique.

4. Système de balayage à faisceau ionique selon l'une quelconque des revendications précédentes, **caractérisé en ce que** le moyen d'absorption d'énergie (7) comporte deux paquets (18) de pièces absorbantes de forme conique mobiles dans des sens opposés transversalement par rapport au centre du faisceau ionique (17).

5. Système de balayage à faisceau ionique selon l'une quelconque des revendications précédentes, **caractérisé en ce que** le système comporte un limiteur périphérique (20) avec des éléments formant diaphragme (21) mobiles entre le volume cible (5) et le moyen d'absorption d'énergie (7).

6. Système de balayage à faisceau ionique selon l'une quelconque des revendications précédentes, **caractérisé en ce que** le système de balayage comporte des diaphragmes périphériques réglables individuellement (19) en forme de diaphragme à iris dans le but de limiter par parties périphériquement le faisceau ionique (3) en relation avec le volume cible.

7. Système de balayage à faisceau ionique selon l'une quelconque des revendications précédentes, **caractérisé en ce qu'**il comporte une table d'examen (22), qui porte le volume cible (5) et peut être déplacée dans deux systèmes de coordonnées dans un plan transversalement par rapport au faisceau ionique (3) pendant une application d'irradiation.

8. Système de balayage à faisceau ionique selon l'une quelconque des revendications précédentes, **caractérisé en ce qu'**il comporte une table d'examen (22), qui porte le volume cible (5) et peut être déplacée dans un sens latéral transversalement par rapport au faisceau ionique (3) pendant une application d'irradiation et comporte des aimants de déflexion (23, 24, 25), qui dévient le faisceau ionique (1) à partir de sa position médiane à la fenêtre de sortie (2) transversalement par rapport à la direction latérale de la table d'examen (22).

9. Système de balayage à faisceau ionique selon l'une quelconque des revendications précédentes, **caractérisé en ce qu'**il comporte une chambre d'ionisation (16) dans le but de mesurer l'intensité de rayonnement, qui est disposée en amont du moyen d'absorption d'énergie (7).

10. Système de balayage à faisceau ionique selon l'une quelconque des revendications précédentes, **caractérisé en ce qu'**il comporte une table d'examen (22), qui porte le volume cible (5) et peut être déplacée dans un sens latéral transversalement par rapport au faisceau ionique (3) pendant une irradiation et comporte un système à portique (27), qui peut tourner autour d'un axe de rotation de portique transversalement par rapport au sens de déplacement latéral de la table d'examen (22).

11. Système de balayage à faisceau ionique avec système à portique (27) pour aligner un faisceau ionique (3) sur un volume cible (5) selon la revendication 10, moyennant quoi le faisceau ionique (3) est guidé vers le système à portique (27) dans l'axe de rotation de portique (28) et peut être aligné sur un volume cible à l'aide des dispositifs optiques magnétiques (23, 24, 25) selon des angles réglables de 0 à 360° dans un plan orthogonalement par rapport à l'axe de rotation de portique (28), de sorte que le faisceau ionique (3) intersecte l'axe de rotation de portique (28) dans un isocentre (29) du système à portique (27), moyennant quoi le système à portique (27) comporte un support du volume cible (30) mobile latéralement dans le sens de l'axe de rotation de portique (28), que le volume cible (5) est disposé en amont de l'isocentre (29) et qu'un moyen d'absorption d'énergie (7), qui est disposé en amont dans le sens radial du système à portique (27), définit une exploration d'un élément de volume dans le sens de la profondeur, le système à portique (27) définit une exploration d'un élément de volume en angle de rotation dans le sens des côtés et le support du volume cible mobile latéralement (30) définit une exploration d'un élément de volume dans le sens longitudinal, et un volume cible d'une quelconque conformation (5) peut être exploré de manière rapportée à son élément de volume au moyen de la combinaison de ces trois moyens d'exploration, moyennant quoi le moyen d'absorption d'énergie (7) comporte des pièces absorbantes de forme conique (13) mobiles transversalement par rapport au centre du faisceau ionique, un moteur linéaire (8) performant pour entraîner les pièces absorbantes de forme conique (13) ainsi qu'un système de commande électronique (14, 34) pour l'entraînement linéaire des pièces absorbantes de forme conique (13).

12. Système de balayage à faisceau ionique avec système à portique selon la revendication 10 ou 11, **caractérisé en ce que** le support du volume cible (30) reste immobile pendant l'irradiation et des aimants de déviation (23, 24, 25) dévient le faisceau ionique (3) dans le plan du portique pendant l'irradiation.

13. Système de balayage à faisceau ionique avec un système à portique selon l'une quelconque des revendications 10 à 12, **caractérisé en ce que** le moyen d'absorption d'énergie (7) comporte des pièces absorbantes de forme conique (13) mobiles tangentiellement par rapport au cercle de révolution du système à portique (27).

14. Système de balayage à faisceau ionique avec un système à portique selon l'une quelconque des revendications 10 à 13, **caractérisé en ce que** le moyen d'absorption d'énergie (7) comporte au moins deux pièces absorbantes de forme conique (13) mobiles, opposées l'une à l'autre, tangentiellement par rapport au cercle de révolution du système à portique (27).

15. Système de balayage à faisceau ionique avec un système à portique selon l'une quelconque des revendications 10 à 14, **caractérisé en ce que** le moyen d'absorption d'énergie (7) comporte des paquets (18) de pièces absorbantes de forme conique mobiles, échelonnées radialement, tangentiellement par rapport au cercle de révolution du système à portique (27).

16. Système de balayage à faisceau ionique avec un système à portique selon l'une quelconque des revendications 13 à 15, **caractérisé en ce qu'**un domaine central du volume cible (5) est disposé autour au moins d'un cinquième du rayon du système à portique en amont de l'isocentre (29), de sorte que le volume cible (9) lui-même ne se trouve pas dans l'isocentre (29).
